# EUROPÄISCHE PATENTSCHRIFT

(11) **EP 2 632 347 B1**
(45) Veröffentlichungstag und Bekanntmachung des Hinweises auf die Patenterteilung: **06.12.2017**
(21) Anmeldenummer: 11785323.4
(22) Anmeldetag: 25.10.2011
(51) Int. Cl.: A61B 17/16, B23B 31/107, A61B 90/00, A61B 17/00

(54) **CHIRURGISCHES INSTRUMENT**
SURGICAL INSTRUMENT
INSTRUMENT CHIRURGICAL

(30) Priorität: 25.10.2010 DE 102010049244
(43) Veröffentlichungstag der Anmeldung: 04.09.2013
(73) Patentinhaber: Dannoritzer Medizintechnik GmbH & Co. KG, 78532 Tuttlingen (DE)
(72) Erfinder: DANNORITZER, Axel, 78532 Tuttlingen (DE)
(74) Vertreter: Daub, Thomas
(86) Internationale Anmeldenummer: PCT/EP2011/005358
(87) Internationale Veröffentlichungsnummer: WO 2012/055530

(56) Entgegenhaltungen:
- EP-A1- 1 938 757
- EP-A1- 1 943 966
- WO-A1-01/66024
- DE-A1- 19 803 998
- GB-A- 2 310 623
- US-A- 2 037 307
- US-A- 4 696 308
- US-A- 5 330 480
- US-A- 5 741 084
- US-A- 5 779 708
- US-A- 5 871 493
- US-A1- 2002 165 549

## Beschreibung

### Stand der Technik

Die Erfindung geht aus von einem Werkzeug, insbesondere zur Verwendung als chirurgischer Bohrer, nach dem Oberbegriff des Anspruchs 1.

Aus der EP 1 943 966 B1 ist bereits ein Werkzeug zur Verwendung als chirurgischer Bohrer mit einem Werkzeuggrundkörper, der zur Verbindung mit einer Werkzeuganbindungseinheit einer Antriebsvorrichtung eine Befestigungseinheit aufweist, die dazu vorgesehen ist, nach einem Lösen des Werkzeuggrundkörpers von der Werkzeuganbindungseinheit zumindest teilweise unbrauchbar zu sein, bekannt.

### Vorteile der Erfindung

Der Erfindung liegt insbesondere die Aufgabe zugrunde, ein Werkzeug bereitzustellen, das mit einer hohen Festigkeit insbesondere in Bezug auf axial wirkende Kräfte an einer Werkzeuganbindungseinheit befestigt werden kann. Die Aufgabe wird insbesondere durch die Merkmale der unabhängigen Patentansprüche gelöst, wobei weitere Ausgestaltungen den Unteransprüchen entnommen werden können.

Die Erfindung geht aus von einem Werkzeug, insbesondere zur Verwendung als chirurgischer Bohrer, mit einem Werkzeuggrundkörper, der zur Verbindung mit einer Werkzeuganbindungseinheit eine Befestigungseinheit aufweist, die dazu vorgesehen ist, nach einem Lösen des Werkzeuggrundkörpers von der Werkzeuganbindungseinheit zumindest teilweise unbrauchbar zu sein.

Es wird vorgeschlagen, dass die Befestigungseinheit zumindest ein Formschlusselement aufweist, das dazu vorgesehen ist, zur axialen Sicherung des Werkzeuggrundkörpers an der Werkzeuganbindungseinheit axiale Zugkräfte aufzunehmen. Dadurch können axiale Kräfte, die auf das Formschlusselement wirken, besonders vorteilhaft auf die Werkzeuganbindungseinheit übertragen werden, wodurch eine besonders feste Verbindung zur axialen Sicherung des Werkzeugs realisiert werden kann. Unter "dazu vorgesehen, nach einem Lösen des Werkzeugs zumindest teilweise unbrauchbar zu sein" soll insbesondere verstanden werden, dass die Befestigungseinheit so ausgebildet ist, dass sie spätestens nach einem mehrmaligen Lösen des Werkzeugs, vorzugsweise aber nach einen erstmaligen Lösen des Werkzeugs, nicht mehr nutzbar ist, und zwar vorzugsweise derart, dass eine erneute axiale Fixierung des Werkzeugs nicht mehr möglich ist. Unter "axialen Zugkräften" soll dabei insbesondere verstanden werden, dass das Formschlusselement dazu vorgesehen ist, Kräfte, die zu einer Zugbelastung des Formschlusselements führen, aufzunehmen und über einen Formschluss an die Werkzeuganbindungseinheit abzuleiten. Unter "Zugkräften" sollen Kräfte verstanden werden, die grundsätzlich zu einer Streckung des Formschlusselements führen würden, sofern das Formschlusselement aus einem elastischen Material ausgebildet wäre. In diesem Zusammenhang soll weiter unter "zur axialen Sicherung vorgesehen" verstanden werden, dass das Formschlusselement dazu vorgesehen ist, auf das Werkzeug wirkende axiale Kräfte in Form einer Zugbelastung auf die Werkzeuganbindungseinheit zu übertragen. Unter "vorgesehen" soll insbesondere speziell ausgestattet und/oder ausgelegt verstanden werden.

Erfindungsgemäß weist die Befestigungseinheit eine Montagerichtung und ein in die Montagerichtung weisendes Auslenkelement auf, das zur beweglichen Anbindung des Formschlusselements an den Werkzeuggrundkörper vorgesehen ist. Dadurch kann eine besonders vorteilhafte Übertragung der Zugkräfte von dem Werkzeuggrundkörper auf das Formschlusselement stattfinden. Das Auslenkelement ist dabei vorzugsweise für eine radiale Auslenkung des Formschlusselements vorgesehen. Unter einer "Montagerichtung" soll dabei insbesondere eine parallel zu einer Rotationsachse des Werkzeugs verlaufende Richtung verstanden werden, die für eine Montage des Werkzeugs an der Werkzeuganbindungseinheit vorgesehen ist. Vorzugsweise soll darunter eine Richtung verstanden werden, die von einer Spitze des Werkzeugs, die mit einer Bohrverzahnung versehen ist, in Richtung eines Endes, an dem der Werkzeuggrundkörper die Befestigungseinheit aufweist, gerichtet ist. Unter einem "Auslenkelement" soll insbesondere ein Element verstanden werden, das dazu vorgesehen ist, das Formschlusselement gegenüber dem Werkzeuggrundkörper auszulenken.
Erfindungsgemäß weisen das Formschlusselement und das Auslenkelement unterschiedliche Erstreckungsrichtungen auf. Dadurch kann eine besonders vorteilhafte Befestigungseinheit realisiert werden. Unter einer "Erstreckungsrichtung" soll dabei insbesondere eine Richtung verstanden werden, die entlang einer Mantelfläche und/oder Oberfläche des Formschlusselements bzw. des Auslenkelements gerichtet ist. Unter unterschiedlichen "Erstreckungsrichtungen" soll insbesondere verstanden werden, dass eine Oberfläche des Formschlusselements und eine Oberfläche des Auslenkelements unter einem Winkel zueinander angeordnet sind.
In einer besonders vorteilhaften Ausgestaltung sind das Formschlusselement und das Auslenkelement in einem spitzen Winkel zueinander angeordnet. Dadurch kann ein besonders vorteilhafter, durch das Formschlusselement bereitgestellter Formschluss erreicht werden, der eine sichere axiale Befestigung des Werkzeugs an der Werkzeuganbindungseinheit ermöglicht. Unter einem "spitzen Winkel" soll dabei insbesondere ein Winkel kleiner als 90 Grad verstanden werden, wobei das Auslenkelement und das Formschlusselement vorzugsweise Oberflächen aufweisen, die den spitzen Winkel einschließen. Vorteilhafterweise ist die Oberfläche des Formschlusselements als eine Anlagefläche zur Befestigung des Werkzeuggrundkörpers vorgesehen und schließt den spitzen Winkel besonders vorteilhaft mit einer Oberfläche des Auslenkelements ein, die in Richtung eines Innenraums des Werkzeuggrundkörpers gerichtet ist.

Zudem ist es vorteilhaft, wenn das Formschlusselement und das Auslenkelement einstückig ausgebildet sind. Dadurch können das Formschlusselement und das Auslenkelement besonders einfach ausgebildet werden. Vorzugsweise sind das Auslenkelement und das Formschlusselement einstückig mit dem Werkzeuggrundkörper ausgebildet.

Weiter wird vorgeschlagen, dass das Formschlusselement um einen Winkel von zumindest 90 Grad gegen das Auslenkelement gebogen ist. Dadurch kann das Formschlusselement besonders einfach realisiert werden. Unter "um einen Winkel von zumindest 90 Grad ausgebogen" soll dabei insbesondere verstanden werden, dass das Formschlusselement durch eine plastische Verformung eines einstückig ausgebildeten Teils hergestellt ist, wobei der Teil, der das Formschlusselement ausbildet, um einen Winkel von zumindest 90 Grad gegenüber einem restlichen Teil, der das Auslenkelement ausbildet, verbogen wird.

In einer besonders vorteilhaften Ausgestaltung weist dabei der Werkzeuggrundkörper ein Ende mit einem Freischnitt auf, der zumindest das Formschlusselement ausbildet. Dadurch können das Formschlusselement und das Auslenkelement besonders einfach hergestellt werden. Vorzugsweise ist das Formschlusselement dabei durch Ausbiegen von einem Teil des Freischnitts hergestellt. Unter einem "Freischnitt" soll dabei insbesondere ein Teil des Werkzeuggrundkörpers verstanden werden, der durch Einbringen von wenigstens einem, vorzugsweise von zwei Einschnitten eine Zunge oder eine Lasche ausbildet, die dazu vorgesehen ist, das Formschlusselement und das Auslenkelement auszubilden. Unter einem "Ende mit einem Freischnitt" soll insbesondere verstanden werden, dass der Freischnitt von dem Ende des Werkzeuggrundkörpers her in den Werkzeuggrundkörper eingebracht ist.

In einer Weiterbildung wird vorgeschlagen, dass der Freischnitt wenigstens teilweise eine Aussparung ausbildet, die zur Übertragung eines Drehmoments vorgesehen ist. Dadurch kann das Werkzeug konstruktiv besonders einfach ausgebildet werden. Insbesondere können dadurch die Aussparung zur Drehmomentübertragung und das Formschlusselement zur axialen Sicherung einstückig ausgeführt und somit miteinander kombiniert werden, wodurch eine besonders einfache Ausbildung der Befestigungseinheit möglich ist.

Außerdem ist es vorteilhaft, wenn die Befestigungseinheit zur Ausbildung einer Steckverbindung vorgesehen ist. Dadurch kann eine einfache Befestigungseinheit realisiert werden.

Weiter wird ein Werkzeug mit einem Werkzeuggrundkörper, der zur drehfesten Verbindung mit einer Werkzeuganbindungseinheit vorgesehen ist und der eine Befestigungseinheit aufweist, die dazu vorgesehen ist, nach einem Lösen des Werkzeuggrundkörpers von der Werkzeuganbindungseinheit zumindest teilweise unbrauchbar zu sein, vorgeschlagen, bei dem die Befestigungseinheit eine Anbindungsaufnahme ausbildet, die zum Aufstecken auf die Werkzeuganbindungseinheit vorgesehen ist. Dadurch kann eine besonders sichere Befestigung des Werkzeugs an der Werkzeuganbindungseinheit erreicht werden. Insbesondere kann dadurch die Befestigungseinheit besonders einfach ausgeführt werden. Unter einer "Anbindungsaufnahme" soll dabei insbesondere ein von dem Werkzeuggrundkörper umschlossener Innenraum verstanden werden, in den ein Aufsteckelement der Werkzeuganbindungseinheit eingebracht werden kann und in dem das Aufsteckelement in montiertem Zustand drehfest angeordnet und axial gesichert ist.

Zudem wird eine Werkzeugbefestigungsvorrichtung zur Verbindung mit einem erfindungsgemäßen Werkzeug vorgeschlagen, die eine Werkzeuganbindungseinheit aufweist, die dazu vorgesehen ist, ein Werkzeug in einer Drehbewegung um eine Rotationsachse rotierend anzutreiben, und die ein Aufsteckelement, das dazu vorgesehen ist, einen Werkzeuggrundkörper wenigstens teilweise zu durchsetzen, sowie zumindest ein Formschlusseingriffselement, das dazu vorgesehen ist, zur Sicherung des Werkzeugs gegen eine axiale Bewegung ein Formschlusselement des Werkzeugs aufzunehmen, aufweist. Dadurch kann eine besonders vorteilhafte und einfache Werkzeugbefestigungsvorrichtung bereitgestellt werden. Unter einem "Formschlusseingriffselement" soll dabei ein Element verstanden werden, das eine Anlagefläche zur Anlage des Formschlusselements des Werkzeugs ausbildet.

Vorzugsweise weist das Formschlusseingriffselement wenigstens eine Anlagefläche für das Formschlusselement auf, die in Bezug auf eine Querschnittsfläche als Schrägfläche ausgebildet ist. Dadurch können axiale Zugkräfte, die auf das Formschlusselement wirken, wenigstens teilweise in radial wirkende Kräfte umgesetzt werden, wodurch ein ungewolltes Herausrutschen des Formschlusselements aus dem Formschlusseingriffselement vorteilhaft verhindert werden kann. Unter einer "Querschnittsfläche" soll eine senkrecht zu der Rotationsachse verlaufende Fläche verstanden werden. Unter einer Ausbildung als "Schrägfläche" soll in diesem Zusammenhang insbesondere verstanden werden, dass die Anlagefläche des Formschlusseingriffselements mit der Querschnittsfläche einen Winkel ungleich Null Grad einschließt. Vorzugsweise beträgt der Winkel etwa 15 Grad. Unter "etwa" im Zusammenhang mit Winkeln soll dabei hier und im Folgenden eine Abweichung von höchstens ± 10 Grad verstanden werden, wobei eine Abweichung von höchstens ± 5 Grad vorteilhaft und eine Abweichung von höchstens ± 1 Grad besonders vorteilhaft ist.

Vorzugsweise bildet das Formschlusseingriffselement wenigstens teilweise einen Hinterschnitt aus. Dadurch kann die Sicherung des Formschlusselements in dem Formschlusseingriffselement besonders einfach realisiert werden. Unter einem "Hinterschnitt" soll dabei insbesondere verstanden werden, dass das Formschlusseingriffselement eine Struktur aufweist, die in Bezug auf die radiale Richtung als ein Hinterschnitt ausgebildet ist, d.h. dass ein Teil der Anlagefläche in Bezug auf die radiale Richtung als eine Ausnehmung ausgebildet ist, in die das Formschlusselement eingreift.

Weiter wird vorgeschlagen, dass das Aufsteckelement zur Ausbildung des Formschlusseingriffselements eine Aussparung aufweist. Dadurch kann das Formschlusseingriffselement besonders einfach ausgebildet werden. Vorzugsweise sind das Aufsteckelement und das Formschlusseingriffselement einstückig ausgebildet.

In einer Weiterbildung wird vorgeschlagen, dass das Aufsteckelement eine Schrägfläche aufweist, die dazu vorgesehen ist, das Formschlusselement des Werkzeugs auszulenken. Dadurch kann das Formschlusselement bei einem Aufstecken des Werkzeugs auf die Werkzeuganbindungseinheit besonders einfach in das Eingriffelelement eingeführt werden, wodurch eine einfache Befestigung des Werkzeugs an der Werkzeugbefestigungsvorrichtung erreicht werden kann.

Zudem ist es vorteilhaft, wenn die Werkzeugbefestigungsvorrichtung eine Antriebsanbindungseinheit aufweist, die zur lösbaren Anbindung an eine Antriebsvorrichtung vorgesehen ist. Dadurch kann die Werkzeugbefestigungsvorrichtung als ein Adapater ausgebildet werden, wodurch bestehende Antriebsvorrichtungen weiter verwendet werden können. Unter einer "Antriebsvorrichtung" soll dabei eine Vorrichtung mit wenigstens einem Antriebsmotor, der dazu vorgesehen ist, das Werkzeug mit einer Drehbewegung zu beaufschlagen, verstanden werden.

Außerdem wird ein System mit einem erfindungsgemäßen Werkzeug und mit einer erfindungsgemäßen Werkzeugbefestigungsvorrichtung vorgeschlagen.

### Zeichnung

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: ein erfindungsgemäßes Werkzeug in einer Aufsicht von oben,
- Fig. 2: das Werkzeug aus Fig. 1 in einer Seitenansicht,
- Fig. 3: das erfindungsgemäße Werkzeug in einer Aufsicht von unten,
- Fig. 4: einen Querschnitt durch eine Befestigungseinheit des Werkzeugs,
- Fig. 5: das Werkzeug in einer perspektivischen Darstellung,
- Fig. 6: eine erfindungsgemäße Werkzeugbefestigungsvorrichtung in einer Seitenansicht,
- Fig. 7: die Werkzeugbefestigungsvorrichtung in einer Aufsicht von oben,
- Fig. 8: eine perspektivische Darstellung der Werkzeugbefestigungsvorrichtung,
- Fig. 9: ein System mit dem Werkzeug aus den Fig. 1 bis 5 und der Werkzeugbefestigungsvorrichtung in montiertem Zustand und
- Fig. 10: das System in einer Aufsicht von unten.

### Beschreibung der Ausführungsbeispiele

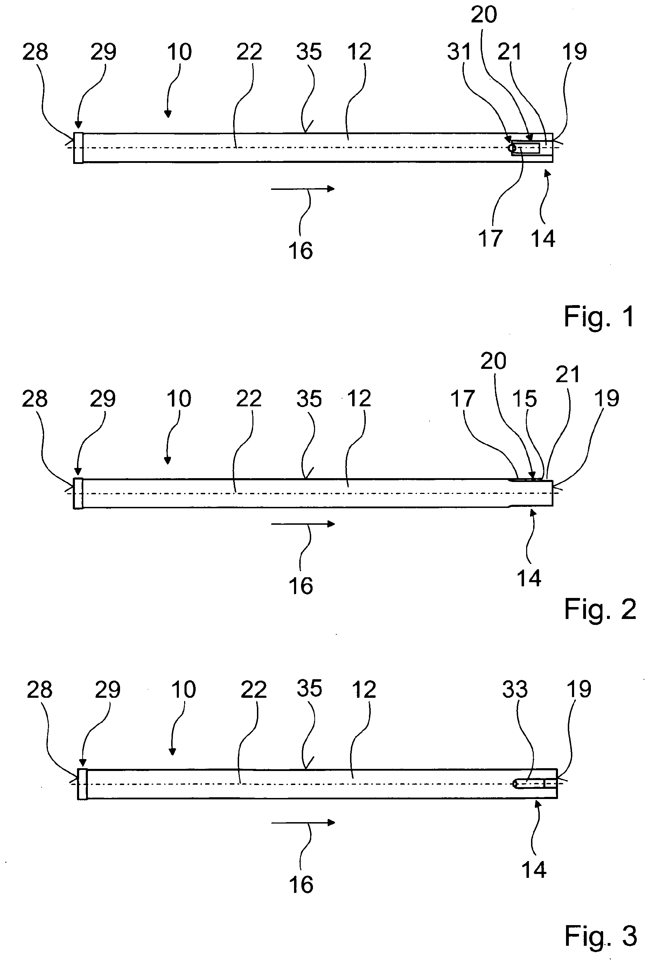

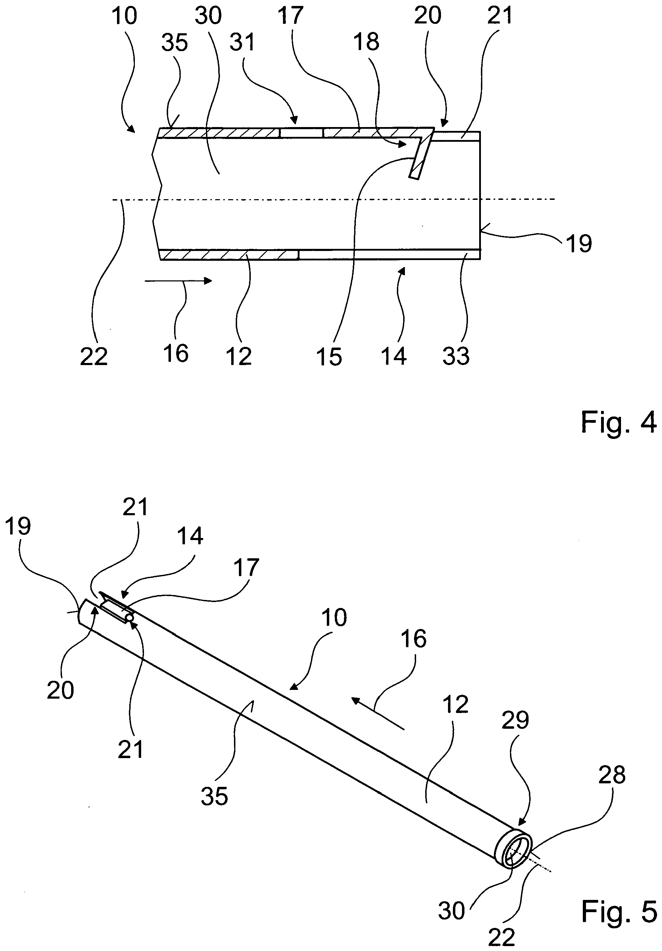

In den Figuren 1 bis 5 ist ein erfindungsgemäßes Werkzeug 10 gezeigt. Das Werkzeug 10 ist zum Einbringen von Bohrungen in Knochen, insbesondere zum Entnehmen von Bohrkernen aus Knochen, vorgesehen. Das Werkzeug 10 umfasst einen Werkzeuggrundkörper 12, der um eine Rotationsachse 22 antreibbar ist.

Der Werkzeuggrundkörper 12 weist eine Spitze 28 auf, die zum Einbringen der Bohrung in den Knochen vorgesehen ist, und ein Ende 19, das zur drehfesten Verbindung mit einer nicht näher dargestellten Antriebsvorrichtung vorgesehen ist. Das Werkzeug 10 bildet damit ein chirurgisches Instrument aus.

An seiner Spitze 28 weist der Werkzeuggrundkörper 12 eine Bohrverzahnung 29 auf, die eine Schleif- oder Schneidvorrichtung ausbildet, mittels der die Bohrung in Knochen eingebracht werden kann. Zur Ausbildung der Bohrverzahnung sind auf die Spitze 28 des Werkzeuggrundkörpers 12 Diamantelemente aufgebracht. Die Diamantelemente sind aus künstlichen Diamanten herstellt. Zum Herausnehmen von Bohrkernen ist der Werkzeuggrundkörper 12 zumindest an der Spitze 28 rohrförmig ausgebildet.

An seinem Ende 19 weist der Werkzeuggrundkörper 12 eine Befestigungseinheit 14 auf. Die Befestigungseinheit 14 ist dazu vorgesehen, den Werkzeuggrundkörper 12 drehfest und gegen eine axiale Bewegung gesichert mit einer Werkzeuganbindungseinheit 13 der Antriebsvorrichtung zu verbinden. Die Befestigungseinheit 14 bildet einen Teil einer Steckverbindung aus. Mittels der Befestigungseinheit 14 ist der Werkzeuggrundkörper 12 durch eine lineare Bewegung entlang der Rotationsachse 22 mit der Werkzeuganbindungseinheit 13 verbindbar. Der Werkzeuggrundkörper 12 ist auch an dem Ende 19 rohrförmig ausbildet. Er ist somit auf die Werkzeuganbindungseinheit 13 aufsteckbar. Eine Richtung der linearen Bewegung definiert dabei eine Montagerichtung 16 für die Befestigungseinheit 14. Die Montagerichtung 16 ist ausgehend von der Spitze 28 in Richtung des Endes 19 gerichtet. Die Bewegung, durch die der Werkzeuggrundkörper 12 mit der Werkzeuganbindungseinheit 13 verbindbar ist, ist entlang dieser Montagerichtung 16 gerichtet.

Das Werkzeug 10 ist als ein Einmalwerkzeug vorgesehen. Um eine mehrfache Verwendung des Werkzeugs 10 zu erschweren oder zu verhindern, ist die Befestigungseinheit 14 so ausgebildet, dass sie nach einem Lösen des Werkzeuggrundkörpers 12 von der Werkzeuganbindungseinheit 13 zumindest teilweise unbrauchbar ist. Die Befestigungseinheit 14 wird dabei beim Lösen des Werkzeuggrundkörpers 12 von der Werkzeuganbindungseinheit 13 derart verformt oder verändert, dass eine erneute drehfeste und axial gesicherte Befestigung des Werkzeuggrundkörpers 12 an der Werkzeuganbindungseinheit 13 nicht mehr möglich ist.

Die Befestigungseinheit 14 umfasst zur axialen Sicherung des Werkzeuggrundkörpers 12 ein Formschlusselement 15, das dazu vorgesehen ist, axiale wirkende Zugkräfte aufzunehmen. Das Formschlusselement 15 ist in Form eines Hakens ausgebildet, der in einem montierten Zustand in ein entsprechendes Formschlusseingriffselement 24 der Werkzeuganbindungseinheit 13 eingreift. Das Formschlusselement 15 sichert dabei den Werkzeuggrundkörper 12 gegen die axiale Bewegung. Die Zugkräfte, die von dem Formschlusselement 15 zur axialen Sicherung des Werkzeuggrundkörpers 12 an der Werkzeuganbindungseinheit 13 aufgenommen werden, sind dabei Kräfte, die entgegen der Montagerichtung 16 wirken.

Zur Anbindung des Formschlusselements 15 an den Werkzeuggrundkörper 12 weist die Befestigungseinheit 14 ein Auslenkelement 17 auf, über das das Formschlusselement 15 mit dem Werkzeuggrundkörper 12 verbunden ist. Das Auslenkelement 17 umfasst ein erstes Ende, das mit dem Werkzeuggrundkörper 12 verbunden ist, und ein zweites Ende, das mit dem Formschlusselement 15 verbunden ist. Ausgehend von dem Ende, das mit dem Werkzeuggrundkörper 12 verbunden ist, erstreckt sich das Auslenkelement 17 in Richtung des Formschlusselements 15.

Das Auslenkelement 17 ist zur beweglichen Anbindung des Formschlusselements 15 an den Werkzeuggrundkörper 12 vorgesehen. Das Formschlusselement 15 und das Auslenkelement 17 bilden einen Teil einer Rasteinheit aus, die dazu vorgesehen ist, die axiale Sicherung des Werkzeuggrundkörpers 12 an der Werkzeuganbindungseinheit 13 zu bewirken. Ausgehend von dem Ende des Auslenkelements 17, das mit dem Werkzeuggrundköper 12 verbunden ist, weist das Auslenkelement 17 in die Montagerichtung 16, also in Richtung des Endes 19. Das Auslenkelement 17 ist in Form einer Lasche oder Zunge ausgebildet, die in Richtung des Endes 19 des Werkzeuggrundkörpers 12 gerichtet ist.

Das Formschlusselement 15 und das Auslenkelement 17 weisen unterschiedliche Erstreckungsrichtungen auf. Das Auslenkelement 17 erstreckt sich im Wesentlichen parallel zu der Rotationsachse 22. In einem Auslieferungszustand ist ein Winkel, den die Erstreckungsrichtung des Auslenkelements 17 mit der Rotationsachse 22 einschließt, in etwa Null Grad. Das Formschlusselement 15 erstreckt sich im Wesentlichen senkrecht zu der Rotationsachse 22. Ein Winkel 18, den die Erstreckungsrichtung des Formschlusselements 15 in dem Auslieferungszustand mit der Hauptrotationsachse 22 einschließt, beträgt in etwa 75 Grad. Der Winkel 18, unter dem das Formschlusselement 15 und das Auslenkelement 17 zueinander angeordnet sind, ist damit als ein spitzer Winkel ausgebildet. Das Auslenkelement 17 weist in die Montagerichtung 16. Das Formschlusselement 15 weist entgegen der Montagerichtung 16.

Das Formschlusselement 15, das Auslenkelement 17 und der Werkzeuggrundkörper 12 sind einstückig ausgebildet. Der gesamte Werkzeuggrundkörper 12 ist rohrförmig ausgebildet. An seinem Ende 19 weist der Werkzeuggrundkörper 12 einen Freischnitt 20 auf, der das Auslenkelement 17 und das Formschlusselement 15 ausgebildet. Der Freischnitt 20 ist dabei durch zwei im Wesentlichen parallel zueinander verlaufende Einschnitte ausgebildet, die von dem Ende 19 her in den Werkzeuggrundkörper 12 eingebracht sind. Das Formschlusselement 15 wird durch Ausbiegen von einem Teil des Freischnitts 20 geformt. Das Formschlusselement 15 ist dabei nach innen ausgebogen. Es ragt somit in einen Innenraum 30, der von dem Werkzeuggrundkörper 12 umschlossen wird, hinein. Das Auslenkelement 17 ist als der Teil des Freischnitts 20 ausgebildet, der im Wesentlichen unverändert bleibt.

In einem Bereich, in dem das Auslenkelement 17 an den Werkzeuggrundkörper 12 angebunden ist, weist das Werkzeug 10 eine Materialschwächung 31 auf. Die Materialschwächung 31 bildet eine Sollbruchstelle aus. Die Materialschwächung 31 ist in dem dargestellten Ausführungsbeispiel durch eine Bohrung ausgebildet, die zwischen den beiden Einschnitten, die den Freischnitt 20 ausbilden, angeordnet ist. Grundsätzlich ist es aber auch denkbar, den Freischnitt 20 mittels aufeinander zulaufender Einschnitte auszubilden, wodurch ebenfalls eine Materialschwächung erreicht werden kann. Zudem ist auch denkbar, dass der Werkzeuggrundkörper 12 in dem Bereich, in dem der Werkzeuggrundkörper 12 in das Auslenkelement 17 übergeht, eine im Vergleich zum übrigen Werkzeuggrundkörper 12 reduzierte Wandstärke aufweist. Die reduzierte Wandstärke kann beispielsweise durch Einbringen einer Nut in diesem Bereich, die vorteilhafterweise durch Stanzen eingebracht wird, realisiert werden.

Durch das Ausbiegen des Formschlusselements 15 bildet der Freischnitt 20 eine Aussparung 21 aus, die sich bis an das Ende 19 des Werkzeuggrundkörpers 12 erstreckt. In die Aussparung 21 kann ein entsprechendes Antriebselement 32 der Werkzeuganbindungseinheit 13 eingreifen, wodurch ein Teil des Freischnitts 20 zur Drehmomentübertragung vorgesehen ist.

Weiter umfasst der Werkzeuggrundkörper 12 eine Aussparung 33, die lediglich zur Drehmomentübertragung vorgesehen ist. Die Aussparung 33 ist axial überschneidend mit dem Freischnitt 20 angeordnet. In Bezug auf die Rotationsachse 22 ist die weitere Aussparung 33 dem Freischnitt 20 gegenüberliegend angeordnet.

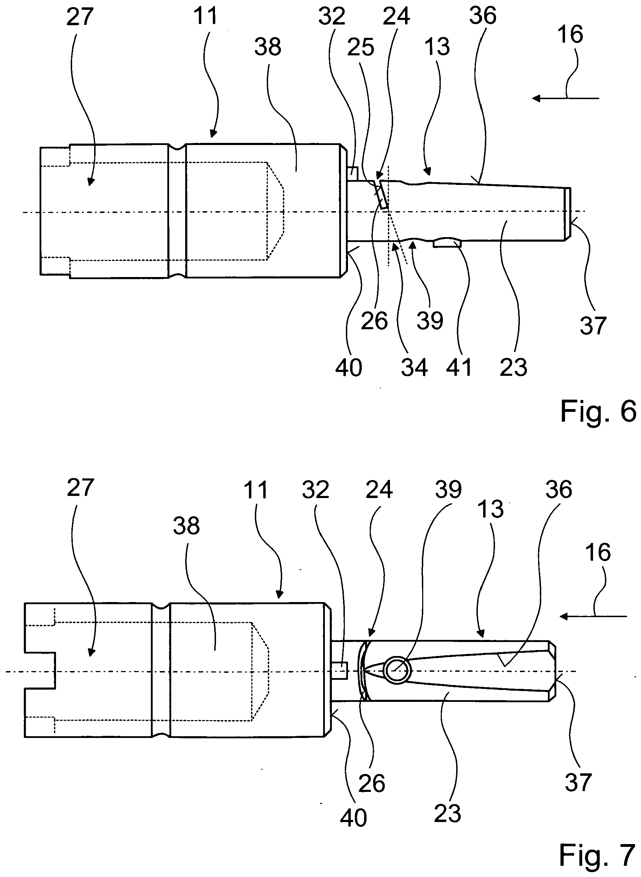

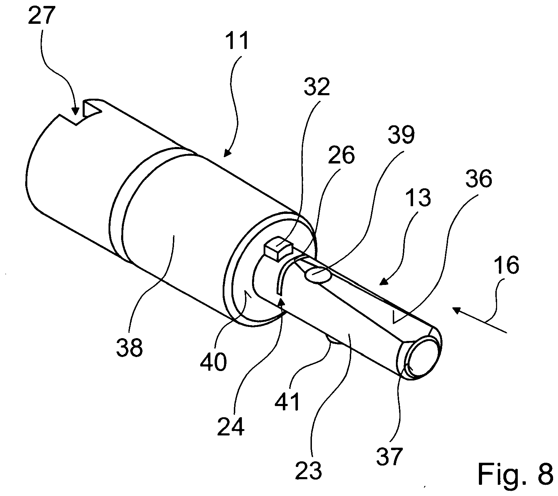

In den Figuren 6 bis 8 ist eine Werkzeugbefestigungsvorrichtung 11 mit der Werkzeuganbindungseinheit 13 zur drehfesten Verbindung und axialen Sicherung des Werkzeugs 10 dargestellt. Die Werkzeuganbindungseinheit 13 umfasst einen Grundkörper 38 und ein mit dem Grundkörper 38 verbundenes Aufsteckelement 23, das in einem montierten Zustand den Werkzeuggrundkörper 12 des Werkzeugs 10 teilweise durchsetzt. Das Aufsteckelement 23 und der Grundkörper 38 sind einstückig ausgebildet. Weiter umfasst die Werkzeuganbindungseinheit 13 das Formschlusseingriffselement 24, das dazu vorgesehen ist, das Formschlusselement 15 des Werkzeuggrundkörpers 12 aufzunehmen.

Das Aufsteckelement 23 weist eine im Wesentlichen zylindrische Grundform auf. Das Aufsteckelement 23 hat dabei einen Außendurchmesser, der zumindest geringfügig kleiner ist als ein Innendurchmesser des Werkzeuggrundkörpers 12. Zur Ausbildung des Formschlusseingriffselements 24, das für das Formschlusselement 15 der Befestigungseinheit 14 des Werkzeuggrundkörpers 12 vorgesehen ist, umfasst das Aufsteckelement 23 eine Aussparung 26. Die Aussparung 26 ist in Form eines Schlitzes ausgebildet. Das Aufsteckelement 23 selbst ist als ein Hohlkörperelement ausgebildet.

Das Formschlusseingriffselement 24 bildet eine Anlagefläche 25 für das Formschlusselement 15 aus. Die Anlagefläche 25 ist dabei in Bezug auf eine Querschnittsfläche, d.h. eine Fläche, die senkrecht zu der Rotationsachse 22 verläuft, als eine Schrägfläche ausgebildet. Die Anlagefläche 25 ist um einen Winkel 34 von etwa 15 Grad gegenüber der Querschnittsfläche verkippt. Der Anlagefläche 25 gegenüberliegend weist das Formschlusseingriffselement 24 eine zweite Fläche auf, die bei der Ausgestaltung der Aussparung 26 in Form eines Schlitzes im Wesentlichen parallel zu der Anlagefläche 25 verläuft. Grundsätzlich kann die Aussparung 26 auch in Form eines Keils ausgebildet sein, wodurch die Anlagefläche 25 und die gegenüberliegende Fläche unter einem Winkel zueinander angeordnet wären.

Das Formschlusseingriffselement 24 bildet dabei in Bezug auf eine radiale Richtung, d.h. eine Richtung, die senkrecht zu der Rotationsachse 22 verläuft, einen Hinterschnitt auf. Die Anlagefläche 25 ist als eine Teilschnittfläche durch das Aufsteckelement 23 ausgebildet. Die Anlagefläche 25 ist damit als eine teilkreisförmige Fläche ausgebildet. Sie umfasst eine Grundlinie, die durch das Aufsteckelement 23 hindurch verläuft, und eine Mantellinie, die durch eine Mantelfläche 35 des Aufsteckelements 23 definiert ist.

Die Grundlinie der Anlagefläche 25 verläuft senkrecht zu der Rotationsachse 22. Von der Grundlinie ausgehende Höhenlinien der Anlagefläche 25 schließen mit der Rotationsachse 22 einen Winkel ungleich 90 Grad ein. Die Anlagefläche 25 ist dabei ausgehend von der Grundlinie in Richtung der Montagerichtung 16 geneigt, wobei die Grundlinie als eine Achse für die Neigung der Anlagefläche 25 angesehen werden kann. Sie bildet somit in radialer Richtung von außen her gesehen einen Hinterschnitt aus, in die das Formschlusselement 15 in montiertem Zustand eingreift. Eine von der Anlagefläche 25 aufgenommene Zugkraft, die über das Formschlusselement 15 übertragen werden kann, bewirkt eine Haltekraft, die ein Herausrutschen des Formschlusselements 15 aus dem Formschlusseingriffselement 24 verhindert oder zumindest erschwert.

Um ein Aufstecken des Werkzeugs 10 zu erleichtern, weist das Aufsteckelement 23 der Werkzeuganbindungseinheit 13 eine Schrägfläche 36 auf, die dazu vorgesehen ist, das Formschlusselement 15 gegenüber dem Werkzeuggrundkörper 12 auszulenken, wenn das Werkzeug 10 an der Werkzeuganbindungseinheit 13 montiert wird. Zur Ausbildung der Schrägfläche 36 weist das Aufsteckelement 23 in einem Teilbereich einen Durchmesser auf, der ausgehend von einer Spitze 37 in Richtung des Grundkörpers 38 stetig zunimmt. Die Schrägfläche 36 ist dabei in Bezug auf die Rotationsachse 22 fluchtend zu dem Formschlusseingriffselement 24 angeordnet.

Zum Lösen des Werkzeugs 10 von der Werkzeuganbindungseinheit 13 weist das Aufsteckelement 23 eine Aussparung 39 auf. Die Aussparung 39 durchsetzt das Aufsteckelement 23 entlang einer Richtung, die senkrecht zu der Rotationsachse 22 orientiert ist, vollständig. Die Aussparung 39 ist dabei im Bereich der Schrägfläche 36 in das Aufsteckelement 23 eingebracht.

Um eine Aufstecktiefe des Werkzeugs 10 zu begrenzen, weist die Werkzeugbefestigungsvorrichtung 11 einen axialen Anschlag 40 auf. Der Grundkörper 38 bildet eine Anschlagfläche für den axialen Anschlag 40 aus. Der Anschlag 40 und das Aufsteckelement 23 sind einstückig mit dem Grundkörper 38 ausgeführt. Zur Ausbildung des Anschlags 40 weist die Werkzeugbefestigungsvorrichtung 11 in einem in axialer Richtung an das Aufsteckelement 23 angrenzenden Bereich einen erhöhten Durchmesser auf.

Zur Übertragung von Drehmomenten umfasst die Werkzeuganbindungseinheit 13 das Antriebselement 32, das dazu vorgesehen ist, in die durch den Freischnitt 20 ausgebildete Aussparung 21 an dem Werkzeuggrundkörper 12 einzugreifen. Weiter umfasst die Werkzeuganbindungseinheit 13 ein Antriebselement 41, das dazu vorgesehen ist, in die Aussparung 33 einzugreifen. Das Antriebselement 32 ist auf der gleichen Seite des Aufsteckelements 23 angeordnet wie das Formschlusseingriffselement 24. Das Antriebselement 41 ist auf der Seite des Aufsteckelements 23 angeordnet, die in Bezug auf die Rotationsachse 22 dem Formschlusseingriffselement 24 gegenüberliegt. Das Antriebselement 32 ist einstückig mit dem Grundkörper 38 ausgeführt. Das Antriebselement 41 ist als ein zusätzlich eingebrachter, fest mit dem Aufsteckelement 23 verbundener Bolzen ausgeführt. Grundsätzlich sind aber beliebige Ausgestaltungen für die Antriebselemente 32, 41 denkbar. Insbesondere kann dabei auch auf das Antriebselement 32 sowie die korrespondierende Aussparung 33 an dem Werkzeuggrundkörper 12 verzichtet werden.

Weiter umfasst die Werkzeugbefestigungsvorrichtung 11 eine Antriebsanbindungseinheit 27, die zur lösbaren Verbindung mit der Antriebsvorrichtung ausgebildet ist. Die Werkzeuganbindungseinheit 13 und die Antriebsanbindungseinheit 27 sind koaxial zueinander ausgerichtet. Die Werkzeuganbindungseinheit 13 und die Antriebsanbindungseinheit 27 sind unterschiedlich ausgestaltet. Die Werkzeugbefestigungsvorrichtung 11 ist damit als ein Adapter ausgebildet, der dazu vorgesehen ist, eine Verbindung zwischen einem Antrieb der Antriebsvorrichtung und dem Werkzeug 10 herzustellen.

Zum Befestigen des Werkzeugs 10 an der Werkzeugbefestigungsvorrichtung 11 wird zunächst der Werkzeuggrundkörper 12 des Werkzeugs 10 auf das Aufsteckelement 23 der Werkzeuganbindungseinheit 13 aufgesteckt. Durch die lineare Bewegung entlang der Montagerichtung 16 wird das Formschlusselement 15 ausgelenkt, wodurch auch das Auslenkelement 17, das zunächst nahezu parallel zu der Mantelfläche 35 des Werkzeuggrundkörpers 12 angeordnet ist, nach außen hin ausgelenkt wird. Sobald das Formschlusselement 15 axial in Höhe des Formschlusseingriffselements 24 ist, greift das Formschlusselement 15 in das Formschlusseingriffselement 24 ein. Durch eine auf das Werkzeug 10 ausgeübte Zugkraft wird das Formschlusselement 15 in vollständigen Eingriff mit dem Formschlusseingriffselement 24 gebracht.

Zum Lösen des Werkzeugs 10 wird das Auslenkelement 17 mit einem geeigneten Werkzeug, das durch die Aussparung 33 in dem Werkzeuggrundkörper 12 und die Aussparung 39 in dem Aufsteckelement 23 hindurch geführt wird, nach außen hin aufgedrückt. Durch das Aufdrücken findet im Bereich der Materialschwächung 31 eine plastische Verformung statt, durch die die Verbindung zwischen dem Werkzeuggrundkörper 12 und dem Auslenkelement 17 geschwächt wird. Spätestens nach einer mehrmaligen Benutzung, vorzugsweise aber nach einer einmaligen Benutzung, ist diese Verbindung soweit geschwächt, dass sie abbricht, wodurch das Werkzeug 10 im Folgenden nicht mehr axial an der Werkzeugbefestigungsvorrichtung 11 befestigt werden kann.

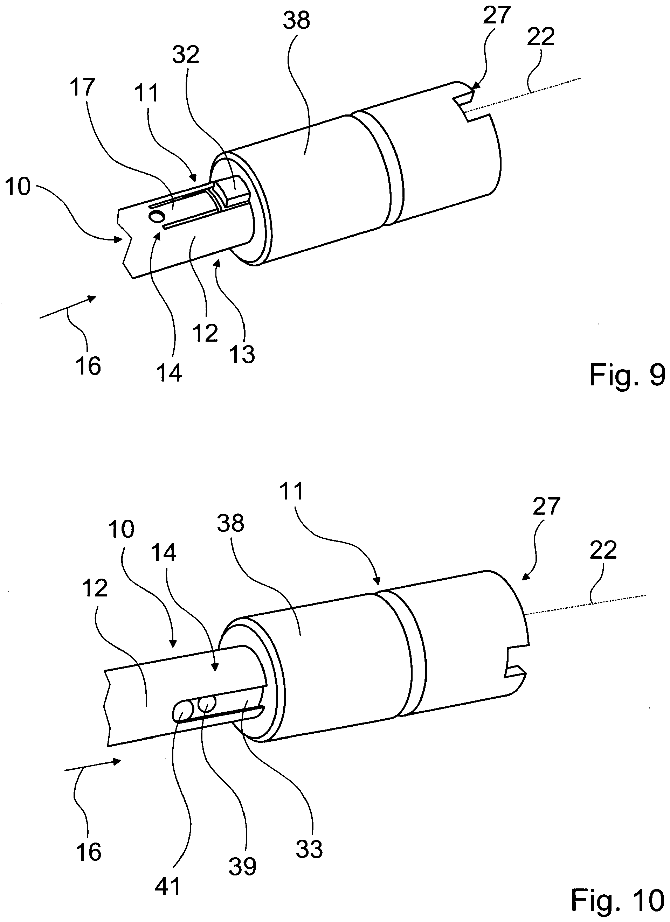

In den Figuren 9 und 10 ist ein System aus der Werkzeugbefestigungsvorrichtung 11 und dem Werkzeug 10 dargestellt. In montiertem Zustand greift das Formschlusselement 15 des Werkzeugs 10 in das Formschlusseingriffselement 24 der Werkzeuganbindungseinheit 13 ein. Das Auslenkelement 17 erstreckt sich in montiertem Zustand wieder nahezu parallel zu der Mantelfläche 35 des Werkzeuggrundkörpers 12. Auf das Werkzeug 10 wirkende Zugkräfte werden über das Auslenkelement 17 und das Formschlusselement 15 auf die Werkzeuganbindungseinheit 13 übertragen. Auf das Auslenkelement 17 und das Formschlusselement 15 wirken dabei ebenfalls Zugkräfte. Eine Fläche des Formschlusselements 15, die der Bohrverzahnung 29 des Werkzeuggrundkörpers 12 zugewandt ist, und die Anlagefläche 25 des Aufsteckelements 23, die dem Anschlag 40 zugewandt ist, bilden dabei die Rastverbindung zur axialen Sicherung des Werkzeugs 10 aus.

In einer nicht näher dargestellten alternativen Ausgestaltung umfasst die Werkzeugbefestigungsvorrichtung 11 eine Mechanik, die dazu vorgesehen ist, das zumindest eine Antriebselement 32, 41 zur drehfesten Anbindung des Werkzeuggrundkörpers 12 unwirksam zu machen. Durch Betätigen der Mechanik wird das entsprechende Antriebselement 32, 41 in dem Aufsteckelement 23 versenkt, wodurch der Werkzeuggrundkörper 12 auf dem Aufsteckelement 23 verdreht werden kann. Durch die Verdrehung wird das Formschlusselement 15 seitlich aus dem Formschlusseingriffselement 24 herausgedreht, wodurch gleichzeitig das Auslenkelement 17 nach außen ausgelenkt wird. Durch die Auslenkung wird die Materialschwächung 31 ebenfalls soweit geschwächt, dass direkt oder bei einem erneuten Einsetzen des Werkzeugs 10 das Auslenkelement 17 mit dem Formschlusselement 15 abbricht. Nach dem Lösen des Werkzeugs 10 von der Werkzeugbefestigungsvorrichtung 11 ist die Befestigungseinheit 14 des Werkzeuggrundkörpers 12 somit unbrauchbar.

**Bezugszeichen**

| | | | |
|---|---|---|---|
| 10 | Werkzeug | | |
| 11 | Werkzeugbefestigungsvorrichtung | 28 | Spitze |
| | | 29 | Bohrverzahnung |
| 12 | Werkzeuggrundkörper | 30 | Innenraum |
| 13 | Werkzeuganbindungseinheit | 31 | Materialschwächung |
| | | 32 | Antriebselement |
| 14 | Befestigungseinheit | 33 | Aussparung |
| 15 | Formschlusselement | 34 | Winkel |
| 16 | Montagerichtung | 35 | Mantelfläche |
| 17 | Auslenkelement | 36 | Schrägfläche |
| 18 | Winkel | 37 | Spitze |
| 19 | Ende | 38 | Grundkörper |
| 20 | Freischnitt | 39 | Aussparung |
| 21 | Aussparung | 40 | Anschlag |
| 22 | Rotationsachse | 41 | Antriebselement |
| 23 | Aufsteckelement | | |
| 24 | Formschlusseingriffselement | | |
| 25 | Anlagefläche | | |
| 26 | Aussparung | | |
| 27 | Antriebsanbindungseinheit | | |

## Patentansprüche

1. Werkzeug zur Verwendung als chirurgischer Bohrer, mit einem Werkzeuggrundkörper (12), der zur Verbindung mit einer Werkzeuganbindungseinheit (13) eine Befestigungseinheit (14) aufweist, die dazu vorgesehen ist, nach einem Lösen des Werkzeuggrundkörpers (12) von der Werkzeuganbindungseinheit (13) zumindest teilweise unbrauchbar zu sein, wobei die Befestigungseinheit (14) zumindest ein Formschlusselement (15) aufweist, das dazu vorgesehen ist, zur axialen Sicherung des Werkzeuggrundkörpers (12) an der Werkzeuganbindungseinheit (13) axiale Zugkräfte aufzunehmen, wobei die Befestigungseinheit (14) eine Montagerichtung (16) und ein in die Montagerichtung (16) weisendes Auslenkelement (17) aufweist, das zur beweglichen Anbindung des Formschlusselements (15) an den Werkzeuggrundkörper (12) vorgesehen ist,
wobei
das Formschlusselement (15) und das Auslenkelement (17) unterschiedliche Erstreckungsrichtungen aufweisen und wobei die Befestigungseinheit (14) eine Anbindungsaufnahme ausbildet, die zum Aufstecken auf die Werkzeuganbindungseinheit (13) vorgesehen ist,
**gekennzeichnet durch**
eine in einem Bereich, in dem das Auslenkelement (17) an den Werkzeuggrundkörper (12) angebunden ist, angeordnete Materialschwächung (31), die eine Sollbruchstelle ausbildet, wobei das Formschlusselement (15) dazu vorgesehen ist, in einem montierten Zustand in ein Formschlusseingriffselement (24) der Werkzeuganbindungseinheit (13) einzugreifen.

2. Werkzeug nach Anspruch 1,
**dadurch gekennzeichnet, dass**
das Formschlusselement (15) und das Auslenkelement (17) unter einem spitzen Winkel zueinander angeordnet sind.

3. Werkzeug zumindest nach Anspruch 1
**dadurch gekennzeichnet, dass**
das Formschlusselement (15) und das Auslenkelement (17) einstückig ausgebildet sind.

4. Werkzeug zumindest nach Anspruch 1,
**dadurch gekennzeichnet, dass**
das Formschlusselement (15) um einen Winkel (18) von zumindest 90 Grad gegen das Auslenkelement (17) gebogen ist.

5. Werkzeug nach einem der vorhergehenden Ansprüche,
**dadurch gekennzeichnet, dass**
der Werkzeuggrundkörper (12) ein Ende (19) mit einem Freischnitt (20) aufweist, der zumindest das Formschlusselement (15) ausbildet.

6. Werkzeug nach einem der vorhergehenden Ansprüche,
**dadurch gekennzeichnet, dass**
die Befestigungseinheit (14) zur Ausbildung einer Steckverbindung vorgesehen ist.

7. System mit einem Werkzeug (10) nach einem der vorhergehenden Ansprüche und mit einer Werkzeugbefestigungsvorrichtung (11) zur Verbindung mit dem Werkzeug (10), mit einer Werkzeuganbindungseinheit (13), die dazu vorgesehen ist, das Werkzeug (10) in einer Drehbewegung um eine Rotationsachse (22) anzutreiben, und die ein Aufsteckelement (23), das dazu vorgesehen ist, einen Werkzeuggrundkörper (12) wenigstens teilweise zu durchsetzen, sowie zumindest ein Formschlusseingriffselement (24), das dazu vorgesehen ist, zur Sicherung des Werkzeugs (10) gegen eine axiale Bewegung ein Formschlusselement (15) des Werkzeugs (10) aufzunehmen, aufweist.

8. System nach Anspruch 7,
**dadurch gekennzeichnet, dass**
das Formschlusseingriffselement (24) wenigstens eine Anlagefläche (25) für das Formschlusselement (15) aufweist, die in Bezug auf eine Querschnittsfläche als Schrägfläche ausgebildet ist.

9. System nach Anspruch 7 oder 8,
**dadurch gekennzeichnet, dass**
das Formschlusseingriffselement (24) wenigstens teilweise einen Hinterschnitt ausbildet.

10. System zumindest nach Anspruch 7,
**dadurch gekennzeichnet, dass**
das Aufsteckelement (23) zur Ausbildung des Formschlusseingriffselements (24) eine Aussparung (26) aufweist.

11. System zumindest nach Anspruch 7,
**dadurch gekennzeichnet, dass**
das Aufsteckelement (23) eine Schrägfläche (36) aufweist, die dazu vorgesehen ist, das Formschlusselement (15) des Werkzeugs (10) auszulenken.

12. System zumindest nach Anspruch 7,
**dadurch gekennzeichnet, dass**
die Werkzeugbefestigungsvorrichtung (11) eine Antriebsanbindungseinheit (27) aufweist, die zur lösbaren Anbindung an eine Antriebsvorrichtung vorgesehen ist.

## Claims

1. Tool for the use as a surgical drill, with a tool base body (12) having, for a connection to a tool connecting unit (13), a fixation unit (14) which is configured to be at least partly unusable subsequently to a detachment of the tool base body (12) from the tool connecting unit (13),
wherein the fixation unit (14) comprises at least one form-fitting element (15) configured to receive axial tensile forces for the purpose of axially securing the tool base body (12) to the tool connecting unit (13),
wherein the fixation unit (14) comprises an assembly direction (16) as well as a deflecting element (17) that points in the assembly direction (16) and is configured for a mobile connection of the form-fitting element (15) to the tool base body (12), wherein the form-fitting element (15) and the deflecting element (17) have differing extension directions and wherein the fixation unit (14) implements a connecting accommodation configured to be plugged onto the tool connecting unit (13),
**characterised by**
a material weakening (31) arranged in a region in which the deflecting element (17) is connected to the tool base body (12) and implementing a predetermined breaking point, wherein the form-fitting element (15) is configured for engaging, in a mounted state, into a form-fitting engagement element (24) of the tool connecting unit (13).

2. Tool according to claim 1,
**characterised in that**
the form-fitting element (15) and the deflecting element (17) are arranged including an acute angle.

3. Tool at least according to claim 1,
**characterised in that**
the form-fitting element (15) and the deflecting element (17) are embodied integrally.

4. Tool at least according to claim 1,
**characterised in that**
the form-fitting element (15) is bent by an angle (18) of at least 90 degrees with respect to the deflecting element (17).

5. Tool according to one of the preceding claims,
**characterised in that**
the tool base body (12) has an end (19) with a cut-out (20) which at least implements the form-fitting element (15).

6. Tool according to one of the preceding claims,
**characterised in that**
the fixation unit (14) is configured to implement a plug connection.

7. System with a tool (10) according to one of the preceding claims and with a tool fixation device (11) for connecting to the tool (10), with a tool connecting unit (13), which is configured to drive the tool (10) about a rotational axis (22) in a rotational movement and which comprises a plug-on element (23) configured to at least partly penetrate a tool base body (12) and comprises at least one form-fitting engagement element (24) configured to accommodate a form-fitting element (15) of the tool (10) for the purpose of securing the tool (10) against an axial movement.

8. System according to claim 7,
**characterised in that**
the form-fitting engagement element (24) comprises at least one abutment surface (25) for the form-fitting element (15), which is embodied as an inclined surface with respect to a cross section surface.

9. System according to claim 7 or 8,
**characterised in that**
the form-fitting engagement element (24) at least partly forms an undercut.

10. System at least according to claim 7,
**characterised in that,**
for implementing the form-fitting engagement element (24), the plug-on element (23) comprises a recess (26).

11. System at least according to claim 7,
**characterised in that**
the plug-on element (23) comprises an inclined surface (36) which is configured to deflect the form-fitting element (15) of the tool (10).

12. System at least according to claim 7,
**characterised in that**
the tool fixating device (11) comprises a drive connection unit (27) which is configured for a releasable connection to a drive device.

## Revendications

1. Outil pour l'usage comme foret chirurgical, avec un corps d'outil de base (12) comportant, pour un raccordement à une unité d'outil de liaison (13), une unité à fixation (14), laquelle est prévue à être, au moins partiellement, inutilisable après un détachement du corps d'outil de base (12) de l'unité d'outil de liaison (13),
l'unité à fixation (14) comprenant au moins un élément de connexion par forme (15) prévu à recevoir des forces de traction axiales pour le bût d'une sécurisation axiale du corps d'outil de base (12) à l'unité d'outil de liaison (13),
l'unité à fixation (14) ayant une direction de montage (16) aussi qu'un élément déflecteur (17) indiquant la direction de montage (16) et prévu pour un raccordement mobile de l'élément de connexion par forme (15) au corps d'outil de base (12),
l'élément de connexion par forme (15) et l'élément déflecteur (17) comportant des directions d'extension différentes, et l'unité à fixation (14) implémentant une accommodation de raccord, laquelle est prévue à être embrochée sur l'unité d'outil de liaison (13),
**caractérisé par**
un affaiblissement de matériau (31) disposé dans une zone, dans laquelle l'élément déflecteur (17) est raccordé au corps d'outil de base (12), l'affaiblissement de matériau (31) implémentant un point de rupture,
l'élément de connexion par forme (15) étant prévu à engrener, en état monté, dans un élément de connexion d'enclenchement par forme (24) de l'unité d'outil de liaison (13).

2. Outil selon la revendication 1, **caractérisé en ce que** l'élément de connexion par forme (15) et l'élément déflecteur (17) sont disposés mutuellement dans un angle aigu.

3. Outil du moins selon la revendication 1, **caractérisé en ce que** l'élément de connexion par forme (15) et l'élément déflecteur (17) sont implémentés intégralement.

4. Outil du moins selon la revendication 1, **caractérisé en ce que** l'élément de connexion par forme (15) est fléchi selon un angle (18) d'au moins 90 degrés vers l'élément déflecteur (17).

5. Outil selon l'une quelconque des revendications précédentes, **caractérisé en ce que** le corps d'outil de base (12) comporte une extrémité (19) avec une découpe (20) implémentant au moins l'élément de connexion par forme (15).

6. Outil selon l'une quelconque des revendications précédentes, **caractérisé en ce que** l'unité à fixation (14) est prévue à implémenter une connexion enfichable.

7. Système avec un outil (10) selon l'une quelconque des revendications précédentes et avec un dispositif à fixation d'outil (11) pour un raccordement à l'outil (10), avec une unité d'outil de liaison (13), laquelle est prévue à entraîner l'outil (10) dans un mouvement rotatif autour d'un axe de rotation (22) et laquelle comporte un élément de fichage (23) prévu à pénétrer un corps d'outil de base (12) au moins partiellement, aussi qu'au moins un élément de connexion d'enclenchement par forme (24) prévu à recevoir un élément de connexion par forme (15) de l'outil (10) pour le bût de sécuriser l'outil (10) contre un mouvement axial.

8. Système selon la revendication 7,
**caractérisé en ce que**
l'élément de connexion d'enclenchement par forme (24) comporte au moins une surface de contact (25) pour l'élément de connexion par forme (15), laquelle est implémentée comme surface inclinée par rapport à une surface de section transversale.

9. Système selon la revendication 7 ou 8,
**caractérisé en ce que**
l'élément de connexion d'enclenchement par forme (24) implémente au moins partiellement une contre-dépouille.

10. Système du moins selon la revendication 7,
**caractérisé en ce que,**
pour implémenter l'élément de connexion d'enclenchement par forme (24), l'élément de fichage (23) comporte un enfoncement (26).

11. Système du moins selon la revendication 7,
**caractérisé en ce que**
l'élément de fichage (23) comporte une surface inclinée (36) prévue à défléchir l'élément de connexion par forme (15) de l'outil (10).

12. Système du moins selon la revendication 7,
**caractérisé en ce que**
le dispositif à fixation d'outil (11) comporte une unité de raccord d'entraînement (27) prévue pour un raccordement détachable à un dispositif d'entraînement.
